Europäisches Patentamt

European Patent Office

Office européen des brevets

(19)

(11) Veröffentlichungsnummer : **0 033 762**
B1

# (12) EUROPÄISCHE PATENTSCHRIFT

(45) Veröffentlichungstag der Patentschrift :
03.08.83

(21) Anmeldenummer : **80106974.1**

(22) Anmeldetag : **12.11.80**

(51) Int. Cl.³ : **C 07 D303/04, C 07 D301/16**

(54) Verfahren zur Herstellung von 1,2-Epoxycyclooctan.

(30) Priorität : **26.01.80 DE 3002793**

(43) Veröffentlichungstag der Anmeldung :
**19.08.81 Patentblatt 81/33**

(45) Bekanntmachung des Hinweises auf die Patenterteilung : **03.08.83 Patentblatt 83/31**

(84) Benannte Vertragsstaaten :
**AT BE CH FR GB IT LI NL SE**

(56) Entgegenhaltungen :
**DE C 962 073**
**FR A 1 399 568**
**US A 2 571 208**
**Methodicum Chimicum (1975), Thieme Verlag, S. 170**

(73) Patentinhaber : **Degussa Aktiengesellschaft**
**Weissfrauenstrasse 9**
**D-6000 Frankfurt am Main 1 (DE)**

(72) Erfinder : **Käbisch, Gerhard, Dr.**
**Palmstrasse 1**
**D-7888 Rheinfelden 5 (DE)**
Erfinder : **Trübe, Rudolf**
**Ernst-Reuter-Strasse 22**
**D-7888 Rheinfelden (DE)**
Erfinder : **Wittmann, Hans**
**Liebenbergstrasse 12**
**D-7888 Rheinfelden 8 (DE)**
Erfinder : **Raupach, Siegfried**
**Langentalstrasse 24**
**D-7888 Rheinfelden (DE)**
Erfinder : **Malitius, Horst**
**Blümleacker 5**
**D-7888 Rheinfelden (DE)**

Verfahren zur Herstellung von 1,2-Epoxycyclooctan

Die Erfindung betrifft ein Verfahren zur Herstellung von 1,2-Epoxycyclooctan durch Umsetzen von Cycloocten mit in situ aus konzentrierter Ameisensäure, die in einer Menge von weniger als 1 Mol pro Mol zu epoxydierender Doppelbindung eingesetzt wird, und aus im Vergleich mit der Ameisensäure weniger konzentriertem Wasserstoffperoxid, das im Überschuss eingesetzt wird, gebildeter Perameisensäure bei erhöhter Temperatur.

Aus der DE-C 962 073 ist bereits ein Verfahren zur Herstellung von 1,2-Epoxycyclooctan durch Umsetzen von Cycloocten mit in situ aus Ameisensäure und Wasserstoffperoxid gebildeter Perameisensäure bekannt. Die Umsetzung erfolgt bei gewöhnlicher oder mässig erhöhter Temperatur. Die Ameisensäure wird in konzentrierter Form in einer Menge von etwa 0,3 bis 2 Mol pro Mol zu epoxydierender Doppelbindung eingesetzt, das Wasserstoffperoxid in einer Konzentration von etwa 35 Gewichtsprozent und in einem geringen Überschuß. Die Umsetzung wird in der Weise vorgenommen, daß entweder sämtliche Reaktionsteilnehmer von Anfang an zusammengemischt werden, oder daß das Cycloocten zusammen mit dem Wasserstoffperoxid vorgelegt und die Ameisensäure zudosiert wird. Das bekannte Verfahren erfordert relativ lange Reaktionszeiten und liefert dennoch nur unbefriedigende Ausbeuten.

Das erfindungsgemäße Verfahren ist nun dadurch gekennzeichnet, daß man das Cycloocten zusammen mit Ameisensäure einer Konzentration von 85 bis 100 Gewichtsprozent in einer Menge von 0,2 bis 0,8 Mol pro Mol zu epoxydierender Doppelbindung vorlegt und bei einer Temperatur zwischen 50 und 65 °C Wasserstoffperoxid einer Konzentration von 30 bis 75 Gewichtsprozent in einer Menge von 1,05 bis 1,55 Mol pro Mol zu epoxydierender Doppelbindung während eines Zeitraumes von mindestens 2 Stunden und höchstens 5 Stunden zudosiert.

Vorzugsweise wird das Wasserstoffperoxid in einer Menge von 1,3 bis 1,5 Mol pro Mol zu epoxydierender Doppelbindung eingesetzt.

Durch das erfindungsgemäße Verfahren kann 1,2-Epoxycyclooctan überraschenderweise innerhalb relativ kurzer Reaktionszeiten bei hohem Umsatz in guter Ausbeute hergestellt werden, obwohl eine im Vergleich mit dem aus der DE-C 962 073 bekannten Verfahren beträchtlich höhere Temperatur angewandt wird.

Um die Temperatur in dem genannten Bereich halten zu können, muss, insbesondere bei grösseren Ansätzen, für Möglichkeiten zu einer ausreichenden Abführung der freiwerdenden Reaktionswärme gesorgt werden. Zweckmässigerweise wird das erfindungsgemässe Verfahren in Abwesenheit von zusätzlichen Lösungsmitteln und von Katalysatoren für die Bildung der Perameisensäure aus der Ameisensäure und dem Wasserstoffperoxid durchgeführt.

Das Wasserstoffperoxid wird während eines Zeitraumes zwischen mindestens 2 Stunden und höchstens 5 Stunden zudosiert. Einschliesslich einer angemessenen Nachreaktionszeit kann dann die für die Epoxydierung insgesamt erforderliche Reaktionszeit auf etwa 8 Stunden oder weniger beschränkt werden. Daraus ergibt sich eine durchaus annehmbare Raum-Zeit-Ausbeute. Bei ausreichend dimensionierten Kühlflächen ist die Gesamtreaktionszeit (= Zudosierungs- und Nachreaktionszeit) umso kürzer, je höher die Konzentrationen des eingesetzten Wasserstoffperoxids und der eingesetzten Ameisensäure sind.

Das erfindungsgemässe Verfahren ist auch in grosstechnischen Produktionsanlagen gefahrlos durchzuführen, wenn einige sicherheitstechnische Massnahmen ergriffen werden. So können z. B. automatische Sicherheitsventile vorgesehen werden, die sich öffnen und das Reaktionsgemisch mit Wasser verdünnen, wenn die Mischorgane ausfallen, die Menge des durch Zersetzung von Wasserstoffperoxid oder Perameisensäure gebildeten Abgases einen vorgegebenen Grenzwert überschreitet oder die Temperatur merklich über 65 °C anzusteigen droht.

Nach beendeter Epoxydierungsreaktion wird im Normalfall die wässrige Phase von der organischen Epoxidphase getrennt. Zur weiteren Reinigung empfiehlt es sich, die organische Phase von darin noch gelösten Wasserstoffperoxid- und Ameisensäure-Anteilen zu befreien. Bevorzugt wird hierzu ein zwei- bis dreimal hintereinander vorgenommenes Auswaschen mit Wasser, wobei das Volumenverhältnis zwischen der gegebenenfalls mit einem inerten Lösungsmittel, wie Ethylacetat, verdünnten, organischen Phase und dem Waschwasser jeweils etwa 10 : 1 bis 2 : 1 beträgt. Das nach dem letzten Auswaschen in der organischen Phase gegebenenfalls enthaltene Lösungsmittel und/oder das darin gelöste Wasser kann leicht, vorzugsweise unter vermindertem Druck, destillativ entfernt werden.

Das nach dem erfindungsgemässen Verfahren erhältliche 1,2-Epoxycyclooctan ist ein wertvolles Zwischenprodukt für Synthesen. Es ist im allgemeinen so rein, dass es für die meisten Zwecke direkt eingesetzt werden kann. Sollte dies in speziellen Fällen erwünscht sein, kann es aber natürlich auch in an sich bekannter Weise, z. B. durch fraktionierte Destillation unter vermindertem Druck, weiter gereinigt werden.

Die nach der Abtrennung der organischen Phase hinterbleibende abgereicherte wässrige Phase kann in an sich bekannter Weise zur Rückgewinnung des enthaltenen Wasserstoffperoxids aufgearbeitet werden.

Das erfindungsgemässe Verfahren soll durch das nachfolgende Beispiel näher erläutert werden :

Beispiel

In einem 1 l-Dreihalskolben mit Rührer, Rück-

flusskühler, Tropftrichter und Thermometer werden vorgelegt

330 g (3,0 Mol) Cycloocten,
42 g (0,9 Mol) Ameisensäure (98 Gewichtsprozent) und
0,3 g des Natriumsalzes der Hydroxyethandiphosphonsäure als Stabilisator für das Wasserstoffperoxid.

Unter intensiver Rührung und Aussenkühlung zur Abführung der freiwerdenden Reaktionswärme werden im Verlaufe von 3 Stunden

340 g (3,9 Mol) Wasserstoffperoxid (40 Gewichtsprozent)

zudosiert.

Während der Zudosierung des Wasserstoffperoxids und einer anschliessenden Nachreaktionszeit von weiteren 3 Stunden wird die Temperatur im Inneren des Kolbens zwischen 55 und 62 °C gehalten. Danach wird die wässrige Phase in einem Scheidetrichter abgetrennt, die organische Phase dreimal hintereinander mit je 50 ml Wasser nachgewaschen und schliesslich im Vakuum (12 Torr) destilliert. Die zwischen 73 und 78 °C übergehende Fraktion (345 g) enthält das 1,2-Epoxycyclooctan in ca. 97 %iger Reinheit (Erstarrungspunkt 48 °C). Das entspricht einer Ausbeute von ca. 92 % der Theorie.

## Ansprüche

1. Verfahren zur Herstellung von 1,2-Epoxycyclooctan durch Umsetzen von Cycloocten mit *in situ* aus konzentrierter Ameisensäure, die in einer Menge von weniger als 1 Mol pro Mol zu epoxydierender Doppelbindung eingesetzt wird, und aus im Vergleich mit der Ameisensäure weniger konzentriertem Wasserstoffperoxid, das im Überschuß eingesetzt wird, gebildeter Perameisensäure bei erhöhter Temperatur, dadurch gekennzeichnet, daß man das Cycloocten zusammen mit Ameisensäure einer Konzentration von 85 bis 100 Gewichtsprozent in einer Menge von 0,2 bis 0,8 Mol pro Mol zu epoxydierender Doppelbindung vorlegt und bei einer Temperatur zwischen 50 und 65 °C Wasserstoffperoxid einer Konzentration von 30 bis 75 Gewichtsprozent in einer Menge von 1,05 bis 1,55 Mol pro Mol zu epoxydierender Doppelbindung während eines Zeitraumes von mindestens 2 Stunden und höchstens 5 Stunden zudosiert.

## Claims

1. A process for the production of 1,2-epoxycyclooctane by the reaction at elevated temperature of cyclooctene with performic acid which is formed *in situ* from concentrated formic acid which is used in a quantity of less than 1 mol per mol of double bond to be epoxidized, and from hydrogen peroxide which is of a lower concentration compared to the formic acid and is used in excess, characterised in that the cyclooctene is introduced together with formic acid of a concentration of from 85 to 100 % by weight in a quantity of from 0.2 to 0.8 mols per mol of double bond to be epoxidized, and hydrogen peroxide of a concentration of from 30 to 75 % by weight is metered in at a temperature of from 50 to 65 °C in a quantity of from 1.05 to 1.55 mols per mol of double bond to be epoxidized over a period of at least 2 hours and at the most 5 hours.

2. A process according to claim 1, characterised in that the hydrogen peroxide is used in a quantity of from 1.3 to 1.5 mols per mol of double bond to be epoxidized.

## Revendications

1. Procédé de préparation du 1,2-époxycyclooctane par réaction du cyclo-octène avec de l'acide performique formé *in situ* à partir d'acide formique concentré, utilisé en proportion inférieure à 1 mole par mole de double liaison à époxyder, et d'eau oxygénée moins concentrée que l'acide formique, et que l'on introduit en excès à température élevée, procédé caractérisé en ce que l'on dispose d'abord le cyclo-octène en commun avec l'acide formique d'une concentration de 85 à 100 % en poids, en proportion de 0,2 à 0,8 mole par mole de double liaison à époxyder, et qu'on y introduit ensuite, à une température comprise entre 50 et 65 °C, du peroxyde d'hydrogène sous forme d'eau oxygénée à la concentration de 30 à 75 % en poids, dans une proportion de 1,05 à 1,55 mole par mole de double liaison à époxyder, en l'espace d'au moins 2 heures et au plus 5 heures.

2. Procédé selon la revendication 1, caractérisé en ce que le peroxyde d'hydrogène est utilisé dans une proportion de 1,3 à 1,5 mole par mole de double liaison à époxyder.